# EUROPEAN PATENT APPLICATION

(11) **EP 2 735 270 A1**
(43) Date of publication of application: **28.05.2014**
(21) Application number: 12815437.4
(22) Date of filing: 22.06.2012
(51) Int. Cl.: A61B 8/00

(54) **ULTRASOUND DIAGNOSTIC APPARATUS AND METHOD FOR DETECTING DETERIORATION OF ULTRASOUND PROBE TRANSDUCER**

(30) Priority: 21.07.2011 JP 2011159551
(71) Applicant: Hitachi Aloka Medical, Ltd., Tokyo 181-8622 (JP)
(72) Inventor: IIMURA,Takashi, Mitaka-shi Tokyo 181-8622 (JP); MORI, Osamu, Mitaka-shi Tokyo 181-8622 (JP)
(74) Representative: MERH-IP Matias Erny Reichl Hoffmann
(86) International application number: PCT/JP2012/066016
(87) International publication number: WO 2013/011800

(57) **Abstract**

In order to provide an ultrasound diagnostic apparatus and method for detecting deterioration of a transducer of an ultrasound probe in which or by which deterioration of a transducer of an ultrasound probe can be correctly detected, an ultrasound diagnostic apparatus comprising an ultrasound probe 12 for transmitting and receiving ultrasonic waves, and a tomographic image constructing part 22 for constructing a tomographic image from reflected echo signals received with the ultrasound probe 12 is provided with a brightness analyzing part 30 for analyzing brightness of a plurality of regions of interest in the tomographic image, and a judging part 34 for judging whether a transducer of the ultrasound probe has deteriorated or not on the basis of analyzed brightness.

## Description

### Technical Field

The present invention relates to an ultrasound diagnostic apparatus for diagnosing a subject using ultrasonic waves, and the present invention relates especially to detection of deterioration of a transducer of an ultrasound probe.

### Background Art

Ultrasound diagnostic apparatuses transmit ultrasonic waves to a subject with an ultrasound probe, receive echo signals of the ultrasonic waves reflected by the subject according to structures of body tissues, construct tomographic images and display them.

In order to detect deterioration of the ultrasound probe, histogram data of pixel values corresponding to a tomographic image of a phantom are first generated. Then, the generated histogram data are compared with histogram data corresponding to a tomographic image of the phantom obtained at the time of shipment of the apparatus as a product or immediately after exchange of the ultrasound probe, and when the difference exceeds a limit of tolerance, deterioration of the ultrasound probe is reported (for example, Patent document 1).

### Prior art reference

### Patent document

Patent document 1: Japanese Patent Unexamined Publication (KOKAI) No. 2002-306478

### Summary of the Invention

### Object to be Achieved by the Invention

In Patent document 1, deterioration state of an ultrasound probe is measured by using histogram data of a tomographic image of a phantom, and therefore it is necessary to perform a measurement using the phantom artificially. Moreover, by the technique of Patent document 1, it is difficult to correctly detect deterioration state of an ultrasound probe due to such factors as secular change of the phantom. In addition, although deterioration of the whole ultrasound probe can be detected by the technique of Patent document 1, it is difficult to detect deterioration of a transducer included in the ultrasound probe.

Therefore, an object of the present invention is to correctly detect deterioration of a transducer of an ultrasound probe.

### Means for Achieving the Object

In order to achieve the object of the present invention, an ultrasound diagnostic apparatus provided with an ultrasound probe for transmitting and receiving ultrasonic waves, a tomographic image constructing part for constructing a tomographic image from reflected echo signals received with the ultrasound probe, a brightness analyzing part for analyzing brightness of a plurality of regions of interest in a tomographic image, and a judging part for judging whether a transducer of the ultrasound probe has deteriorated on the basis of analyzed brightness.

There is also contemplated a method for detecting deterioration of a transducer of an ultrasound probe, which is provided with the step of constructing a tomographic image from reflected echo signals received with the ultrasound probe, the step of analyzing brightness of a plurality of regions of interest in the tomographic image, and the step of judging whether a transducer of the ultrasound probe has deteriorated or not on the basis of analyzed brightness.

### Effect of the Invention

According to the present invention, deterioration of a transducer of an ultrasound probe can be correctly detected.

### Brief Description of the Drawings

Fig. 1 is a block diagram showing the configuration of the ultrasound diagnostic apparatus of the present invention.
Fig. 2 shows a mode for setting regions of interest according to the present invention.
Fig. 3 shows a graph of brightness in regions of interest according to the present invention.
Fig. 4 shows a graph of brightness in regions of interest according to the present invention.
Fig. 5 shows a mode for displaying alert information according to the present invention.
Fig. 6A shows implementation modes of Embodiment 2 according to the present invention.
Fig. 6B shows implementation modes of Embodiment 2 according to the present invention.
Fig. 7 shows implementation mode of Embodiment 3 according to the present invention.

### Modes for Carrying out the Invention

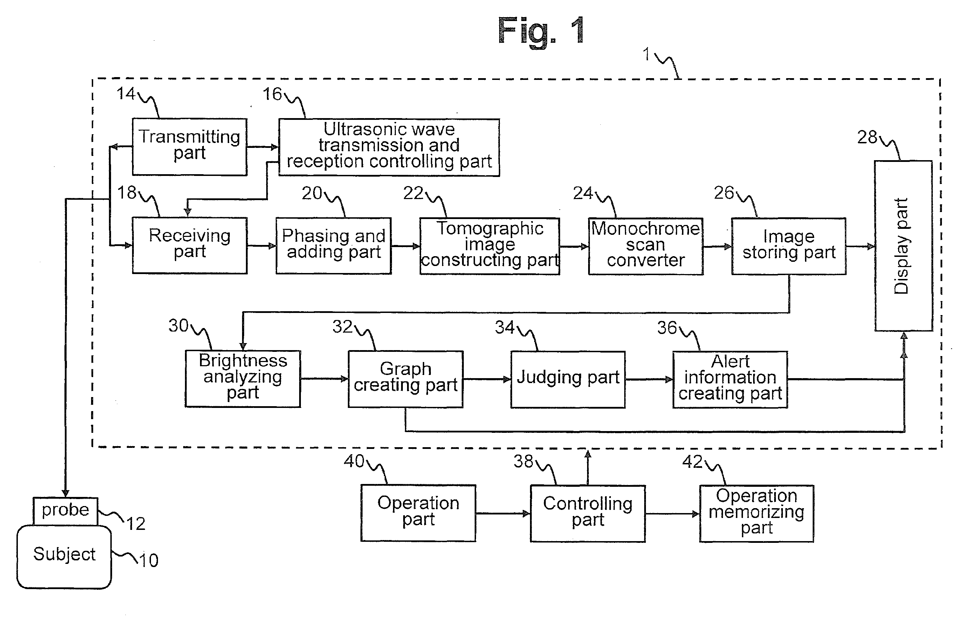

The ultrasound diagnostic apparatus according to the present invention will be explained with reference to the drawings. Fig. 1 is a block diagram showing the configuration of the ultrasound diagnostic apparatus of the present invention.

### Embodiment 1

As shown in Fig. 1, the ultrasound diagnostic apparatus is provided with an ultrasound probe 12 which is used by being contacted with a subject 10, a transmitting part 14 for repeatedly transmitting ultrasonic waves to the subject 10 with a time interval via the ultrasound probe 12, a receiving part 18 for receiving reflected time series echo signals generated from the subject 10, a transmission and reception controlling part 16 for controlling the transmitting part 14 and the receiving part 18, and a phasing and adding part 20 for phasing and adding the reflected echo signals received by the receiving part 18.

The apparatus is also provided with a tomographic image constructing part 22 for constructing a tomographic image, for example, a monochrome (shade) tomographic image, of the subject 10 from RF signal frame data sent from the phasing and adding part 20, a monochrome scan converter 24 for converting output signals of the tomographic image constructing part 22 into an image suitable for display on a display part 28, an image storing part 26 for memorizing the tomographic image with time information, and the display part 28 for displaying the tomographic image, measurement information, and so forth.

The apparatus is also provided with a brightness analyzing part 30 for analyzing brightness of a region of interest in the tomographic image, a graph creating part 32 for creating a brightness graph from brightness values analyzed by the brightness analyzing part 30, a judging part 34 for judging whether a transducer of the ultrasound probe 12 has deteriorated on the basis of the brightness graph created by the graph creating part 32, and an alert information creating part 36 for creating alert information on the basis of the result of the judgment.

The apparatus is further provided with a controlling part 38 for controlling the aforementioned components of the body 1 of the ultrasound diagnostic apparatus, an operation part 40 for giving directions to the controlling part 38, and an operation memorizing part 42 for memorizing the operation information inputted through the operation part 40. The operation part 40 consists of a keyboard having various keys, trackball, and so forth.

Hereafter, the ultrasound diagnostic apparatus will be explained in detail. The ultrasound probe 12 is provided with a plurality of transducers, and has a function of transmitting and receiving ultrasonic waves to and from the subject 10 through the transducers. The transmitting part 14 has a function of generating a wave transmission pulse for driving the ultrasound probe 12 to generate ultrasonic waves and setting a convergent point of the transmitted ultrasonic waves at a certain depth. The receiving part 18 amplifies the reflected echo signals received by the ultrasound probe 12 with a predetermined gain to generate RF signals, i.e., received wave signals. The phasing and adding part 20 inputs the RF signals amplified by the receiving part 18, controls the phases of them, and forms ultrasonic beams for one or a plurality of convergent points to generate RF signal frame data.

The tomographic image constructing part 22 receives the RF signal frame data sent from the phasing and adding part 20, and performs signal processings such as gain correction, log compression, detection, contour enhancement, and filter processing to obtain tomographic image data. The monochrome scan converter 24 is provided with an analog to digital converter for converting the tomographic image data sent from the tomographic image constructing part 22 into digital signals and a controller, and reads out the obtained tomographic image data by television synchronization. The image storing part 26 memorizes a plurality of tomographic images formed by conversion with the monochrome scan converter 24 together with time information.

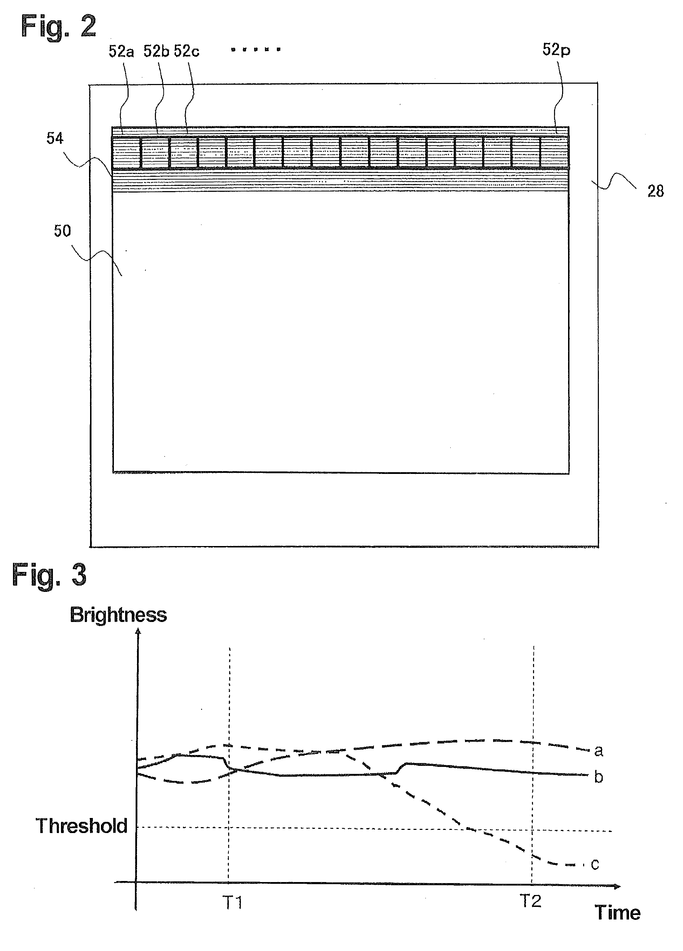

A tomographic image memorized by the image storing part 26 is read out, and displayed on the display part 28. An operator sets a plurality of regions of interest in the tomographic image displayed on the display part 28 by using the operation part 40. The operation part 40 can be used as a region of interest setting part. Specifically, as shown in Fig. 2, the operator sets a plurality of regions of interest 52a to 52p in a tomographic image 50 displayed on the display part 28 by using the operation part 40. The tomographic image 50 read out from the image storing part 26 is a linear type (rectangular) tomographic image constructed by using a linear type probe as the ultrasound probe 12.

The tomographic image 50 read out from the image storing part 26 is a tomographic image constructed when the ultrasound probe 12 is in an aerial radiation state. That is, it is a tomographic image constructed when the ultrasound probe 12 is not contacted with the subject 10. The image storing part 26 periodically memorizes tomographic images constructed when the ultrasound probe 12 is in an aerial radiation state together with time information by periodically transmitting and receiving ultrasonic waves when the ultrasound probes 12 is in an aerial radiation state at a time not during ultrasonic imaging such as the time of activating, inactivating or resting the ultrasound diagnostic apparatus. For example, the image storing part 26 memorizes a tomographic image constructed when the ultrasound probe 12 is in an aerial radiation state once a day. The operation memorizing part 42 memorizes ultrasound imaging conditions (conditions for transmission and reception, conditions for display on the screen). The operation memorizing part 42 may memorize an operation procedure used for the operation part 40. Since the imaging conditions are read out from the operation memorizing part 42, and the tomographic image constructing part 22 constructs a tomographic image using the imaging conditions, the image storing part 26 can memorize a plurality of tomographic images constructed with the same imaging conditions. Therefore, it can be secured that tomographic images obtained always under the same imaging conditions are recorded in the image storing part 26.

The operator selects positions of a plurality of the regions of interest 52a to 52p by rolling the trackball of the operation part 40, and determines the positions of a plurality of the regions of interest 52a to 52p by pressing an enter key of the keyboard of the operation part 40. Further, the operator selects sizes of a plurality of the regions of interest 52a to 52p by rolling the trackball of the operation part 40, and determines the sizes of the plurality of the regions of interest 52a to 52p by pressing an enter key of the keyboard of the operation part 40.

When the ultrasound probe 12 is in an aerial radiation state, ultrasonic waves are reflected at the surface of the ultrasound probe 12, and received by the ultrasound probe 12. That is, a multiple reflection region 54 is generated at a position of a small depth of the tomographic image 50 (depth of several centimeters or smaller). As the multiple reflection region 54, a multiple reflection region of triple reflection or multiple reflection of further higher degree can also be theoretically contemplated.

The operator sets a plurality of the regions of interest 52a to 52p by using the operation part 40 at positions where the multiple reflection region 54 is generated, i.e., positions of a small depth (depth of several centimeters or smaller). A plurality of the regions of interest 52a to 52p are set at the same depths for the depth direction, and with equal intervals and without gaps on the tomographic image 50 for the scanning direction so that all the transducers of the ultrasound probe 12 can be covered. The operator can also arbitrarily set the height (depth direction) and width (scanning direction) of a plurality of the regions of interest 52a to 52p by using the operation part 40. As described above, a plurality of the regions of interest 52a to 52p are set within the multiple reflection region 54 of the tomographic image 50. For example, if the width of a plurality of the regions of interest 52a to 52p for the scanning direction is set to be narrow, the number of a plurality of the regions of interest increases, and therefore brightness can be analyzed in more detail.

For example, if the ultrasound probe 12 is constructed with 128 channels of transducers, each region of interest among a plurality of the regions of interest 52a to 52p is covered by 8 channels of the transducers. As described above, for the scanning direction, a plurality of the regions of interest 52a to 52p are set without any gaps.

Further, a plurality of the regions of interest 52a to 52p can also be collectively set as a group of regions of interest. A plurality of the regions of interest 52a to 52p arranged in one row along the scanning direction are considered as a group of the regions of interest. That is, in the group of the regions of interest, a plurality of the regions of interest 52a to 52p are set with equal intervals and without any gaps for the scanning direction. By specifying the depth for the group of the regions of interest with the operation part 40, the operator can set a plurality of the regions of interest 52a to 52p at once.

The controlling part 38 may automatically set a plurality of the regions of interest 52a to 52p at positions of a small depth of the tomographic image 50 where the multiple reflection region 54 is generated. Specifically, since the depth of the positions of small depth where the multiple reflection region 54 is generated is several centimeters or less, the controlling part 38 sets a plurality of the regions of interest 52a to 52p at a depth in the range of several centimeters or smaller (for example, 2 to 3 cm) for the depth direction, and with equal intervals and without any gaps in the tomographic image 50 for the scanning direction. In the above Embodiment, the controlling part 38 sets a plurality of regions of interest 52a to 52p along the scanning direction, and the number of the regions of interest is 16. However, the controlling part 38 can also arbitrarily set the number of the regions of interest according to a number of channels inputted through the operation part 40. For example, if it is intended to cover each region of interest with 4 channels of transducers, the operator inputs the number of channels as 4 through the operation part 40. The controlling part 38 sets 32 regions of interest according to 4 channels inputted through the operation part 40. Further, for example, if it is intended to cover each region of interest with 2 channels of transducers, the operator inputs the number of channels as 2 through the operation part 40. The controlling part 38 sets 64 regions of interest according to 2 channels inputted through the operation part 40.

The operation memorizing part 42 memorizes positions and sizes of a plurality of the regions of interest (group of regions of interest) set at positions of a small depth of the tomographic image 50 where the multiple reflection region 54 is generated. By appropriately reading out positions and sizes of a plurality of the regions of interest (group of the regions of interest) memorized in the operation memorizing part 42, the controlling part 38 can set a plurality of regions of interest (group of regions of interest) within the multiple reflection region 54 also for a tomographic image obtained at a different time point. That is, a plurality of the same regions of interest (group of the regions of interest) can be set for the tomographic images 50 obtained at different time points.

The brightness analyzing part 30 calculates average brightness for each of a plurality of the regions of interest 52a to 52p. Average brightness is a value calculated by adding brightness values of pixels within a region of interest, and dividing the sum with the number of the pixels within the region of interest. As described above, average brightness is calculated for each of a plurality of the regions of interest 52a to 52p. The calculated average brightness values of a plurality of the regions of interest 52a to 52p are memorized in a storage part (not shown in the drawings) with time and date (time information).

The graph creating part 32 creates a brightness graph on the basis of present and past average brightness values calculated for a plurality of the regions of interest 52a to 52p by the brightness analyzing part 30 and memorized in the storage part. Specifically, such a brightness graph as shown in Fig. 3 is created for a plurality of the regions of interest 52a to 52p. In the brightness graph, the vertical axis indicates brightness, and the horizontal axis indicates time (day). The brightness graph based on the average brightness values calculated for a plurality of the regions of interest 52a to 52p created by the graph creating part 32 is displayed on the display part 28.

Hereafter, explanation will be made for a brightness graph for only three of the regions of interest 52a, 52b, and 52c for simplicity of indication in Fig. 3 and simplicity of the explanation. The brightness graph for the region of interest 52a is represented by the curve a, the brightness graph for the region of interest 52b is represented by the curve b, and the brightness graph for the region of interest 52c is represented by the curve c.

Since the analysis is performed by using the tomographic image 50 constructed by periodically (for example, once a day) transmitting and receiving ultrasonic waves when the ultrasound probe 12 is in an aerial radiation state, the brightness graph is also periodically (for example, once a day) renewed whenever the tomographic image 50 is newly obtained.

As shown in Fig. 3, although the brightness values for the regions of interest 52a and 52b are substantially constant, the brightness value for the region of interest 52c decreases. If a transducer of the ultrasound probe 12 deteriorates, brightness of the tomographic image 50 obtained with the transducer that has deteriorated decreases, and the tomographic image 50 is blackened. Therefore, the operator can see that the transducer of the ultrasound probe 12 used for obtaining the tomographic image 50 of the region of interest 52c has deteriorated.

The judging part 34 compares the brightness value calculated for a region of interest with a predetermined threshold value of brightness to judge whether a transducer of the ultrasound probe 12 corresponding to the region of interest has deteriorated or not.

When the brightness value for the region of interest 52a exceeds the threshold value, the judging part 34 judges that the transducer of the ultrasound probe 12 corresponding to the region of interest 52a normally works. When the brightness value for the region of interest 52c is smaller than the threshold value, the judging part 34 judges that the transducer of the ultrasound probe 12 corresponding to the region of interest 52c has deteriorated.

Specifically, at the time point T1 in the graph shown in Fig. 3, the brightness values for the regions of interest 52a, 52b and 52c are larger than the threshold value, and therefore the judging part 34 judges that the transducers of the ultrasound probe 12 normally work. At the time point T2, the brightness values for the regions of interest 52a and 52b are larger than the threshold value, but the brightness value for the region of interest 52c is smaller than the threshold value. Therefore, the judging part 34 judges that the transducer of the ultrasound probe 12 corresponding to the region of interest 52c has deteriorated.

The operator can arbitrarily set the threshold value (brightness) by using the operation part 40.

The judging part 34 judges whether a transducer of the ultrasound probe 12 corresponding to a region of interest has deteriorated on the basis of comparison of brightness values calculated for regions of interest. For the comparison of the brightness values calculated for the regions of interest, for example, ratio to average brightness value calculated for the regions of interest is used.

The judging part 34 calculates a ratio of brightness value for the region of interest 52a to the average brightness value for the regions of interest other than the region of interest 52a, and if the ratio is in a predetermined range (for example, 0.8 to 1.2), the judging part 34 judges that the transducer of the ultrasound probe 12 corresponding to the region of interest 52a normally works. The judging part 34 calculates a ratio of brightness value for the region of interest 52a to the average brightness value for the regions of interest other than the region of interest 52a, and if the ratio is out of the predetermined range (smaller than 0.8 or larger than 1.2), the judging part 34 judges that a transducer of the ultrasound probe 12 corresponding to the region of interest 52a has deteriorated.

Specifically, in the case of the graph shown in Fig. 3, if the brightness value for the region of interest 52c is compared with the average brightness value for the regions of interest other than 52c for the time point T1, the ratio of the brightness value for the region of interest 52c to the average brightness value for the regions of interest other than 52c is in the predetermined range, and therefore the judging part 34 judges that a transducer of the ultrasound probe 12 corresponding to the region of interest 52c normally works. At the time point T2, the ratio of the brightness value for the region of interest 52c to the average brightness value for the regions of interest other than 52c is out of the predetermined range, and therefore the judging part 34 judges that a transducer of the ultrasound probe 12 corresponding to the region of interest 52c has deteriorated.

The judging part 34 can also judge whether a transducer of the ultrasound probe 12 corresponding to a region of interest has deteriorated or not on the basis of rate of change of the brightness value calculated for the region of interest. The rate of change of the brightness value calculated for a region of interest is, for example, inclination of a brightness curve.

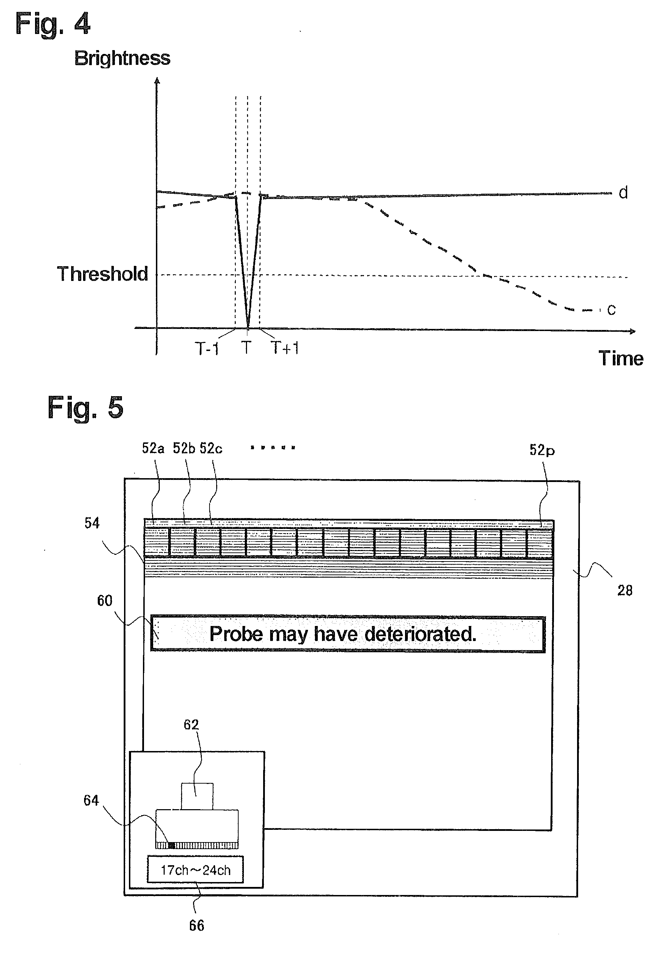

As represented by the brightness curve c for the region of interest 52c shown in Fig. 4, a transducer of the ultrasound probe 12 usually gradually deteriorates. That is, the rate of change of brightness value is smaller than a predetermined value.

When the rate of change of the brightness value for a region of interest is not smaller than the predetermined value, such a large rate of change may be caused by failure of the transducer of the ultrasound probe 12 due to impact, or adhesion of fouling such as ultrasonic jelly on the ultrasonic wave transmission and reception surface of the ultrasound probe 12. When the rate of change of the brightness value for a region of interest is not smaller than the predetermined value, the display part 28 may display a message "Please remove fouling on the ultrasonic wave transmission and reception surface of the ultrasound probe 12".

When the rate of change of the brightness value for the region of interest 52c is not smaller than the predetermined value, the operator removes fouling on the ultrasonic wave transmission and reception surface of the ultrasound probe 12, and then the judging part 34 performs the judgment again for the brightness in the region of interest.

In the case shown in Fig. 4, in the period from the time point T1 to the time point T, the rate of change of the brightness value for the region of interest 52d is large, and exceeds the predetermined value.

The operator removes fouling on the ultrasonic wave transmission and reception surface of the ultrasound probe 12, and then measures the brightness in the region of interest 52d at the time point T+1. In the period from the time point T-1 to the time point T+1, the rate of change of the brightness value for the region of interest 52d is small, and does not exceed the predetermined value. Therefore, the judging part 34 judges that a transducer of the ultrasound probe 12 corresponding to the region of interest 52d normally works.

Although not shown in the drawing, when the rate of change of the brightness value for the region of interest 52d is also large for the period from the time point T-1 to the time point T+1, and it is larger than the predetermined value, the judging part 34 judges that a transducer of the ultrasound probe 12 corresponding to the region of interest 52d is out of order.

The operator can choose any of these judgment methods for the judgments based on a threshold value, ratio, or rate of change explained above for use in the judging part 34. Further, priorities may be given to the judgment methods used in the judging part 34, and the judgment may be performed by using the methods in the order of higher priority to lower priority. For example, the judging part 34 can perform the judgment using a threshold value, and then perform the judgment using rate of change.

When it is judged that a transducer of the ultrasound probe 12 has deteriorated, the alert information creating part 36 creates alert information (alarm) on the basis of the judgment by the judging part 34, and the display part 28 displays the alert information.

When it is judged by the judging part 34 that a transducer of the ultrasound probe 12 has deteriorated, the alert information creating part 36 creates a message 60, "Probe may have deteriorated", as the alert information. The display part 28 displays the message 60.

The alert information creating part 36 may also create an ultrasound probe model 62 to be displayed on the display part 28 together with the alert information. The ultrasound probe model 62 schematically shows the ultrasound probe 12. The transducers are schematically indicated at one end of the ultrasound probe model 62.

For example, when each of a plurality of regions of interest 52a to 52p is covered by the transducers of 8 channels, the brightness value for the region of interest 52c is smaller than the threshold value, and therefore the judging part 34 judges that one or more transducers of the ultrasound probe 12 corresponding to the region of interest 52c have deteriorated, the alert information creating part 36 displays the channels 66 (17th to 24th channels) of the transducers corresponding to the region of interest 52c on the display part 28.

Further, in order to make it possible to intuitively understand which transducers among the transducers of the ultrasound probe 12 have deteriorated, the alert information creating part 36 can also make the display part 28 display a deterioration mark 64 on the transducers of the ultrasound probe model 62 corresponding to the region of interest 52c.

As described above, according to the present invention, an ultrasound diagnostic apparatus provided with an ultrasound probe 12 for transmitting and receiving ultrasonic waves, a tomographic image constructing part 22 for constructing a tomographic image from reflected echo signals received with the ultrasound probe, a brightness analyzing part 30 for analyzing brightness of a plurality of regions of interest in the tomographic image, and a judging part 34 for judging whether a transducer of the ultrasound probe has deteriorated or not on the basis of analyzed brightness. Therefore, deterioration of a transducer of the ultrasound probe 12 can be correctly detected.

In addition, when it is judged by the judging part 34 that any of the transducers of the ultrasound probe 12 has deteriorated, the controlling part 38 can control the apparatus through the ultrasonic wave transmitting and receiving part 16 so that ultrasonic waves cannot be transmitted and received from and to the ultrasound probe 12. There can be thereby invited a situation that the operator comes to be unable to use the ultrasound diagnostic apparatus with the ultrasound probe 12 that has deteriorated, and must call a service staff of the ultrasound diagnostic apparatus. Therefore, ultrasound diagnosis can always be performed by using the ultrasound probe 12 that does not deteriorate.

### Embodiment 2

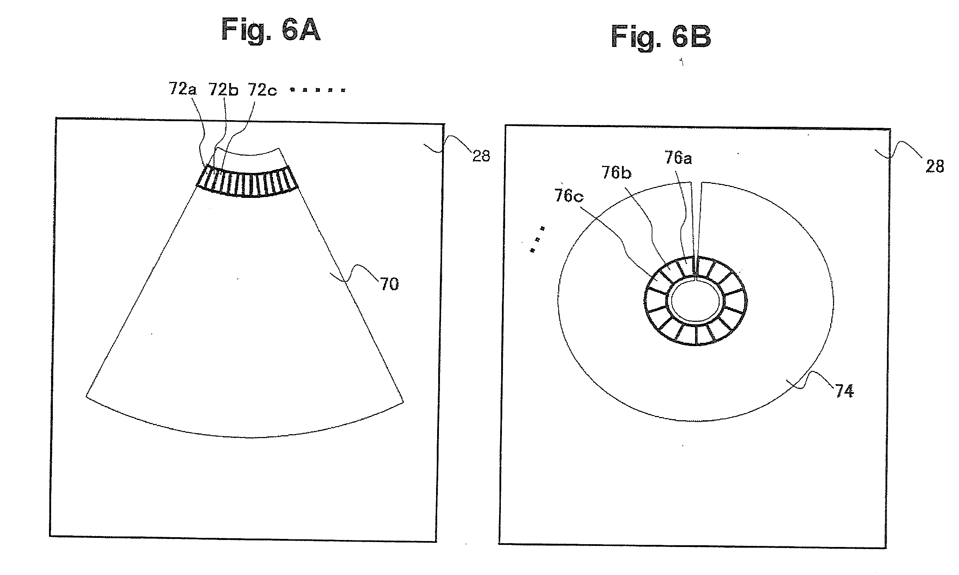

The apparatus of Embodiment 2 will be explained below with reference to Fig. 6A and Fig. 6B. The apparatus of Embodiment 2 differs from that of Embodiment 1 in that a plurality of regions of interest are set according to the type of the ultrasound probe 12. According to the type (shape) of the ultrasound probe 12, the shape and arrangement of the regions of interest are determined.

In Embodiment 1, the ultrasound probe 12 is a linear type one, and therefore the rectangular regions of interest 52a to 52p are arranged on one straight line along the scanning direction.

In this Embodiment, as shown in Fig. 6A, the ultrasound probe 12 is a convex type one (fan shape), and therefore the tomographic image 50 is a convex type one (fan shape). Therefore, fan-shaped regions of interest 72a ... are arranged in a fan shape along a curve for the scanning direction according to the shape of the tomographic image 50. A plurality of the regions of interest 72a ... are set at positions where multiple reflection is generated, i.e., positions of a small depth (depth of several centimeters or smaller). A plurality of the regions of interest 72a ... are set at the same depths for the depth direction, and with equal intervals and without gaps on the tomographic image 50 for the scanning direction, so that all the transducers of the ultrasound probe 12 can be covered.

Further, a plurality of the regions of interest 72a ... can also be collectively set as a group of regions of interest. A plurality of the regions of interest 72a ... arranged along a curve in a fan shape for the scanning direction are considered to be a group of the regions of interest. By specifying the depth for the group of the regions of interest with the operation part 40, the operator can set a plurality of the regions of interest 72a ... at once.

Further, as shown in Fig. 6B, when the ultrasound probe 12 is a radial type one (circular shape), the tomographic image 50 is a radial type one (circular shape). Therefore, fan-shaped regions of interest 72a ... are arranged in a circular shape along a circle for the scanning direction according to the shape of the tomographic image 50. A plurality of the regions of interest 76a ... are set at positions where multiple reflection is generated, i.e., positions of a small depth (depth of several centimeters or smaller). A plurality of the regions of interest 76a ... are set at the same depths for the depth direction, and with equal intervals and without gaps on the tomographic image 50 for the scanning direction, so that all the transducers of the ultrasound probe 12 can be covered.

Further, a plurality of the regions of interest 76a ... can also be collectively set as a group of regions of interest. A plurality of the regions of interest 76a ... arranged along a circle in a circular shape for the scanning direction are considered as a group of the regions of interest. By specifying the depth for the group of the regions of interest with the operation part 40, the operator can set a plurality of the regions of interest 76a ... at once.

According to this Embodiment, the shape and arrangement of the regions of interest are set according to the type (shape) of the ultrasound probe 12, deterioration of the transducers of the ultrasound probe 12 can be correctly detected with regions of interest suitable for the type (shape) of the ultrasound probe 12.

### Embodiment 3

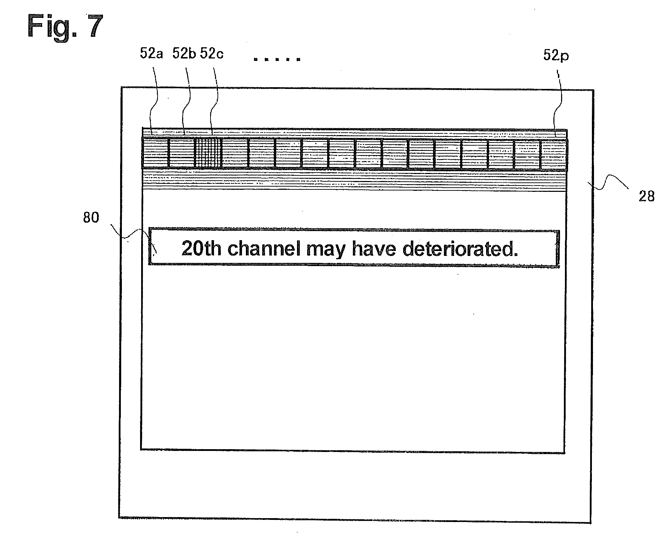

The apparatus of Embodiment 3 will be explained below with reference to Fig. 7. The apparatus of Embodiment 3 differs from those of Embodiments 1 and 2 in that the regions of interest are set in a number corresponding to the number of channels of the transducers of the ultrasound probe 12, and the judging part 34 specifies channel of a transducer of the ultrasound probe 12 that has deteriorated.

For example, as shown in Fig. 7, when the brightness value for the region of interest 52c is smaller than the threshold value, and the judging part 34 judges that a transducer of the ultrasound probe 12 corresponding to the region of interest 52c has deteriorated, the region of interest 52c is further divided. Since each region of interest is covered by 8 channels of the transducers, the region of interest 52c is divided into eight, which corresponds to the number of channels.

Then, the brightness analyzing part 30 calculates average brightness values for all the divided regions of interest. The judging part 34 compares the brightness values calculated for the divided regions of interest with a predetermined threshold value for brightness, and judges whether the transducers of the ultrasound probe 12 corresponding to the regions of interest have deteriorated or not.

If brightness value for a certain divided region of interest is smaller than the threshold value, the judging part 34 judges that the transducer of the ultrasound probe 12 corresponding to the divided region of interest has deteriorated, and specifies the channel of the transducer of the ultrasound probe 12.

The alert information creating part 36 indicates which channel of the transducer has deteriorated. When it is judged by the judging part 34 that the transducer of the 20th channel in the ultrasound probe 12 has deteriorated, the alert information creating part 36 creates a message 80, "20th channel may have deteriorated" as the alert information.

According to this Embodiment, the region of interest is divided into a number corresponding to the number of channels of the transducers of the ultrasound probe 12, and therefore the operator can specify the channel of the transducer of the ultrasound probe 12 that has deteriorated.

### Denotation of Reference Numerals

10 ... Subject, 12 ... ultrasound probe, 14 ... transmitting part, 16 ... ultrasonic wave transmission and reception controlling part, 18 ... receiving part, 20 ... phasing and adding part, 22 ... tomographic image constructing part, 24 ... monochrome scan converter, 26 ... image storing part, 28 ... display part, 30 ... brightness analyzing part, 32 ... graph creating part, 34 ... judging part, 36 ... alert information creating part, 38 ... controlling part, 40 ... operation part, 42 ... operation memorizing part,

## Claims

1. An ultrasound diagnostic apparatus comprising
an ultrasound probe for transmitting and receiving ultrasonic waves,
a tomographic image constructing part for constructing a tomographic image from reflected echo signals received with the ultrasound probe,
a brightness analyzing part for analyzing brightness of a plurality of regions of interest in the tomographic image, and
a judging part for judging whether a transducer of the ultrasound probe has deteriorated or not on the basis of analyzed brightness.

2. The ultrasound diagnostic apparatus according to claim 1, wherein the tomographic image is a tomographic image constructed when the ultrasound probe is in an aerial radiation state.

3. The ultrasound diagnostic apparatus according to claim 1, wherein a plurality of the regions of interest are set in a multiple reflection region of the tomographic image.

4. The ultrasound diagnostic apparatus according to claim 3, wherein a plurality of the regions of interest are set with equal intervals and without gaps for the scanning direction.

5. The ultrasound diagnostic apparatus according to claim 1, wherein the judging part compares a brightness value calculated for a region of interest with a predetermined threshold value of brightness to judge whether a transducer of the ultrasound probe corresponding to the region of interest has deteriorated or not.

6. The ultrasound diagnostic apparatus according to claim 1, wherein the judging part calculates a ratio of brightness value for a region of interest to average brightness value for regions of interest other than the foregoing region of interest to judge whether a transducer of the ultrasound probe corresponding to the region of interest has deteriorated or not.

7. The ultrasound diagnostic apparatus according to claim 1, wherein the judging part judges whether a transducer of the ultrasound probe corresponding to a region of interest has deteriorated or not on the basis of rate of change of brightness value calculated for the region of interest.

8. The ultrasound diagnostic apparatus according to claim 1, which comprises a display part for displaying alert information when a transducer of the ultrasound probe has deteriorated.

9. The ultrasound diagnostic apparatus according to claim 1, wherein, when it is judged by the judging part that any of the transducers of the ultrasound probe has deteriorated, the ultrasound probe is controlled so as to be unable to transmit and receive ultrasonic waves.

10. The ultrasound diagnostic apparatus according to claim 1, wherein a plurality of regions of interest are set according to type of the ultrasound probe.

11. The ultrasound diagnostic apparatus according to claim 1, wherein the regions of interest are divided into regions in a number corresponding to the number of channels of the transducers of the ultrasound probe, and the judging part specifies channel of a transducer of the ultrasound probe that has deteriorated.

12. A method for detecting deterioration of a transducer of an ultrasound probe, which comprises
the step of constructing a tomographic image from reflected echo signals received with the ultrasound probe,
the step of analyzing brightness of a plurality of regions of interest in the tomographic image, and
the step of judging whether a transducer of the ultrasound probe has deteriorated or not on the basis of analyzed brightness.
